# DEMANDE DE BREVET EUROPEEN

(11) **EP 2 277 450 A1**
(43) Date de publication de la demande: **26.01.2011**
(21) Numéro de dépôt: 09166267.6
(22) Date de dépôt: 23.07.2009
(51) Int. Cl.: A61B 8/00, A61B 6/04, A61B 8/08, A61B 19/00, A61G 13/10, A61N 5/10

(54) **Dispositif de maintien d'au moins une sonde à ultrason**

(71) Demandeur: Siemens Schweiz AG, 8047 Zürich (CH)
(72) Inventeur: Cloutot, Laurent, 8956, Killwangen (CH)
(74) Mandataire: Fischer, Michael

(57) **Abrégé**

La présente invention décrit un dispositif de maintien d'au moins une sonde (E) à ultrason adaptée à la visualisation d'une zone limitée d'un sujet mobile et déformable (P), comprenant un support mobile (S_{E}) dans et par rapport à une ouverture (S_{T}) d'un plan (T) contre lequel une partie superficielle du sujet est appuyée, le dit support mobile maintenant solidairement au moins une partie émettrice/réceptrice de la sonde de façon à la rendre librement mobile par diverses poussées mécaniques exercées par des pressions variables provenant de la partie superficielle en direction de l'ouverture.

## Description

La présente invention concerne un dispositif de maintien d'au moins une sonde à ultrason selon le préambule de la revendication 1, ainsi que des aspects liés à son utilisation.

Actuellement, un objectif majeur réside dans la visualisation de parties internes telles qu'in-vivo chez un sujet de type humain ou animal ou un mannequin dit aussi « fantôme ». En particulier, cet objectif est crucial dès lors qu'une intervention telle qu'une irradiation locale radiothérapeutique ou technologiquement équivalente en dépend afin de pouvoir localement irradier une zone limitée incluant des cellules à détruire tout en préservant de l'irradiation de tissus et cellules saines avoisinés aux cellules malsaines.

Actuellement par exemple, lors d'une séance de radiothérapie sur des cellules cancéreuses telle qu'une tumeur ou un carcinome, un dispositif annexe à l'installation de radiothérapie permet de prendre des prises de vue de type radiographique afin de visualiser et localiser ladite tumeur in-vivo dans le référentiel de l'installation de radiothérapie, ceci aux fins de contrôler que le faisceau irradiant concentre bien son énergie sur la tumeur. Ce procédé à base d'irradiation nuisible (rayons X) n'est toutefois pas sans danger pour un patient déjà affaibli par un cancer et ne permet pas une visualisation ainsi qu'une localisation d'une cible à intervalles fréquents et donc a fortiori en temps réel lors de séances d'irradiation. De tels procédés sont aussi volumineux, coûteux et nécessitent de multiples interventions humaines pour leur positionnement, leur fonctionnement et leur maintenance. Un but de la présente invention est ainsi de pouvoir localiser un volume de cellules in-vivo, telles que des cellules cancéreuses, de façon la plus continue possible, en particulier à intervalles fréquents durant une séance d'irradiation desdites cellules.

Un autre but de l'invention est aussi de pouvoir localiser un volume de cellules in-vivo, telles que des cellules cancéreuses, de façon la plus exacte possible, afin en particulier qu'une irradiation des dites cellules reste dynamiquement centrée sur le volume tout en épargnant des tissus sains périphériques. Ceci est d'autant plus important car il est inévitable qu'un patient (appuyé ou allongé sur une table comme surface de référence) n'est jamais immobile, et ses organes internes sont aussi mobiles, comme basiquement par exemple lors de ses cycles de respiration.

Enfin, un problème alternatif de l'invention concerne le fait que la visualisation et la localisation d'une tumeur par exemple doit être effectuée en préservant la santé mais aussi le confort et la quiétude du patient afin de lui éviter tout stress complémentaire à un diagnostic voire une thérapie plus lourde et astreignante. Ainsi, toute irradiation invasive ou du moins nuisible, en supplément à celle liée à une possible radiothérapie ou une thérapie similaire, ainsi que l'usage de méthodes de maintien du patient, de marquage ou autre contrainte infligée au patient doit être minimisé, et idéalement éliminé.

L'invention propose ainsi un dispositif approprié à résoudre cet ensemble de problèmes et décrit au travers de la revendication 1.

A cet effet, l'invention prévoit un dispositif de maintien d'au moins une sonde à ultrason adaptée à la visualisation d'une zone limitée d'un sujet mobile et déformable tel qu'un patient. Le dispositif comprend un support mobile dans et par rapport à une ouverture d'un plan (table, lit muni d'un évidement en surface) contre lequel une partie superficielle du sujet est appuyée, le dit support mobile maintenant solidairement au moins une partie émettrice/réceptrice de la sonde de façon à la rendre librement mobile par diverses poussées mécaniques exercées par des pressions variables provenant de la partie superficielle en direction de l'ouverture.

De part l'intégration avantageuse d'une sonde à ultrasons, il est ainsi possible de visualiser et localiser la zone limitée (volume de cellules tumorales par exemple) de façon continue et en temps réel, même chez un patient lourdement malade et affaibli, car les ultrasons n'ont pas d'effets nocifs et affaiblissants connus. Si le patient bouge par rapport à une table où il est allongé, le support mobile initialement disposé pour permettre d'inclure la zone visée et son voisinage dans son champ de vision (eux-mêmes en possible mouvement, par exemple par de simples raisons respiratoire, de flux sanguin ou d'autres aspects dynamiques in-vivo) assure un déplacement naturel de la sonde afin de toujours cadrer la zone visée dans son champ de vision. Cette unique prédisposition de la sonde peut-être réglée en début d'une phase de visualisation grâce à un opérateur, afin d'assurer une visualisation et localisation sans faille a posteriori.

Les sondes à ultrasons actuelles permettent une vision bi- ou tridimensionnelle et donc, une fois que la zone limitée (tumeur) est localisée, il est possible de recalculer sa position par la simple connaissance du déplacement naturel de la sonde dans son support mobile par rapport au plan d'appui du patient (fixe ou du moins spatialement connu dans le référentiel de l'installation annexe de diagnostic/thérapie). Ladite localisation est donc fort exacte spatialement et absolument dynamique temporellement.

Un ensemble de sous-revendications présente également des avantages de l'invention.

Des exemples de réalisation et d'application sont fournis à l'aide de figures décrites :
- Figure 1: dispositif de maintien d'au moins une sonde à ultrason selon l'invention,
- Figure 2: schéma d'utilisation du dispositif selon l'invention pour contrôler un positionnement d'appareillage annexe.

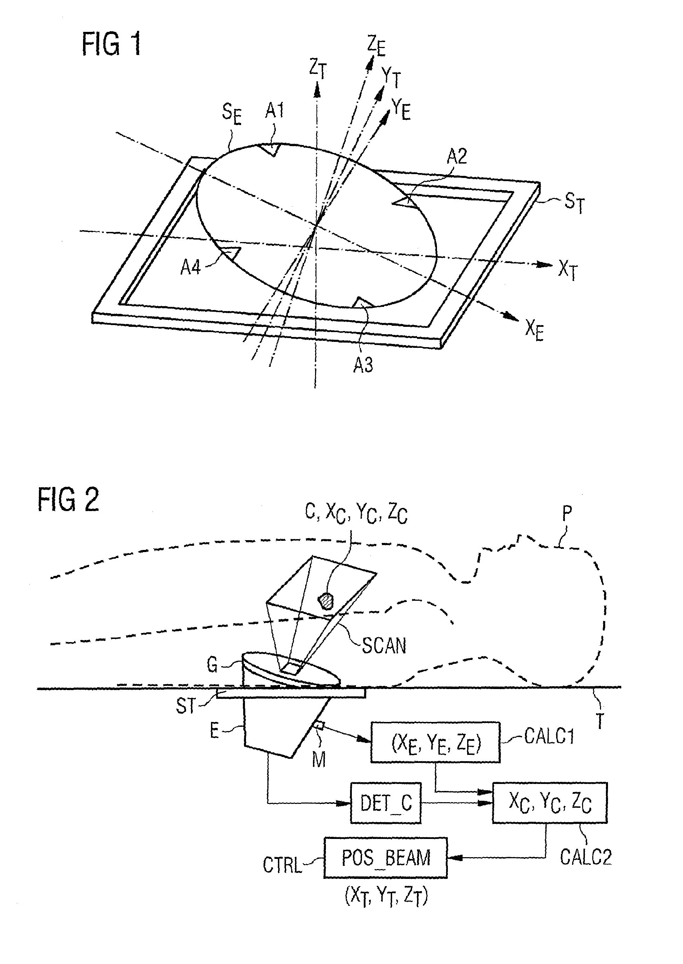

La figure 1 présente un dispositif de maintien d'au moins une sonde à ultrason adaptée à la visualisation d'une zone limitée d'un sujet mobile et déformable. La sonde et le sujet sont ici non représentés pour des raisons de clarté, mais le seront à la figure 2.
Ledit dispositif comprend un support mobile (Se) dans et par rapport à une ouverture (St), comme ici un cadre évidé dans un plan (table, lit ou support d'appui) contre lequel une partie superficielle du sujet est appuyée, le dit support mobile maintenant solidairement au moins une partie émettrice/réceptrice de la sonde de façon à la rendre librement mobile par diverses poussées mécaniques exercées par des pressions variables provenant de la partie superficielle du sujet en direction de l'ouverture.

La sonde peut être maintenue dans le support mobile par des moyens de serrage A1, A2, A3, A4, le support mobile étant lui-même principalement mu par un système de rotation libre par rapport à l'ouverture-cadre comparablement à moyen de rotation d'un compas marin s'adaptant à tourner vers un cap horizontal tout en compensant une gite vertical au navire. Le référentiel spatial X_{T}, Y_{T}, Z_{T} de l'ouverture-cadre S_{T} étant connue de façon absolue, la position initiale et la rotation postérieure libre et instantanée de la sonde peut ainsi être déduite de la mesure en continue de la position d'axes X_{E}, Y_{E}, Z_{E} du référentiel orthonormé de la sonde ou du support mobile dans le référentiel X_{T}, Y_{T}, Z_{T} de l'ouverture-cadre S_{T}. Cette mesure peut être exécutée par divers moyens métrologique usuellement électroniques ou opto-électroniques de nos jours. La sonde délivrant ensuite les coordonnées de la zone limitée (tumeur localisée), il en découle que la position de la tumeur est immédiatement obtenue dans le référentiel X_{T}, Y_{T}, Z_{T} de l'ouverture-cadre S_{T} et donc dans tout autre référentiel s'y rattachant comme le référentiel spatial d'une installation annexe de radiothérapie et donc par extension de la cible de son faisceau de radiation vers la tumeur visée.

La figure 2 présente à cet effet un schéma d'utilisation possible du dispositif selon l'invention pour contrôler CTRL un positionnement d'appareillage annexe tel qu'un faisceau de radiation POS_BEAM d'une installation radio-thérapeutique. Un patient P en tant que sujet mobile et déformable est couché sur une table T muni de l'ouverture-cadre S_{T}. Il est important que la sonde à ultrasons E maintenue dans l'ouverture-cadre soit décalée du chemin du faisceau d'une installation annexe de radiothérapie afin de ne pas être endommagée si la sonde à ultrasons fonctionne en même temps que le faisceau radio-thérapeutique. Ceci est possible en plaçant l'ouverture-cadre sur une partie de la table non ciblée par le faisceau et en inclinant la sonde initialement par rapport à la verticale de la table. Il est aussi possible de disposer la sonde sur un autre plan que celui de la table, par exemple sur une paroi latérale tout en assurant l'appui de la sonde contre une surface du patient propice à guider les ultrasons vers la zone à visualiser comprenant la tumeur.

L'invention prévoit qu'au moins un film de gel acoustique G est disposé entre la sonde E et la partie de contact superficiel entre le patient et ladite sonde afin d'évacuer un interstice d'air sur un canal d'ultrasons entre la sonde et la partie superficielle.

Alternativement, la partie émettrice/réceptrice de la sonde est alimentée par un flux de gel acoustique G provenant d'un réservoir de distribution dudit gel permettant une canalisation directe des ultrasons entre la sonde et la partie superficielle, ladite canalisation étant ainsi rendue libre d'interstice d'air.

Egalement, il est aussi possible que la partie émettrice/réceptrice de la sonde soit recouverte d'une enceinte de gel acoustique G à géométrie variable assurant l'appui de la partie superficielle et permettant une canalisation directe des ultrasons entre la sonde et la partie superficielle, ladite canalisation étant ainsi rendue libre d'interstice d'air. Alternativement, au moins une surface de l'ouverture peut comprendre une enceinte de gel acoustique G à géométrie variable assurant l'appui de la partie superficielle et permettant une canalisation directe des ultrasons entre la sonde et la partie superficielle, ladite canalisation étant rendue libre d'interstice d'air. L'enceinte G peut ainsi être une forme élastomère à géométrie variable et ayant une impédance acoustique minimisant des pertes acoustique entre une entrée/sortie d'ultrasons de la sonde et de la partie superficielle, idéalement par une valeur d'impédance avoisinée à celle de la partie superficielle.

Le dispositif selon l'invention prévoit que, par entrainement du support mobile S_{E}, l'ouverture est positionnable sur au moins une dimension du plan. Ceci permet une plus grande flexibilité de positionnement de la sonde en face de la zone à visualiser et localiser. Enfin, le support mobile SE est inclinable (ici librement) autour d'une normale au plan d'appui T, afin de suivre librement des mouvements quelconques d'appui du patient.

Afin de pouvoir calculer ces mouvements du patient ainsi que le déplacement potentiel et donc la position X_{C}, Y_{C}, Z_{C} de la zone visualisée C à localiser (tumeur) pendant une acquisition d'image SCAN de la sonde à ultrasons E, le support mobile SE comprend des premiers moyens de détermination et de calcul CALC1 de sa position X_{E}, Y_{E}, Z_{E} (ou celle de la sonde) en termes de translation et d'orientation dans un référentiel tridimensionnel connu lié au plan T. La position X_{C}, Y_{C}, Z_{C} de la zone visualisée C est délivrée par la mesure de la sonde ou du moins un module de localisation spatiale associé DET_C et peut donc à l'aide d'un moyen de calcul CALC2, similaire aux premiers moyens de calcul CALC1, être aussi déterminée dans le référentiel tridimensionnel connu lié au plan T.

Après connaissance de la position de X_{C}, Y_{C}, Z_{C} de la zone visualisée C à localiser (tumeur), celle-ci peut être transmise sous des coordonnées adaptées X_{T}, Y_{T}, Z_{T} dans un référentiel propre à un appareillage annexe de positionnement POS_BEAM tel que celui pour contrôler CTRL spatialement la cible d'un faisceau d'irradiation radio-thérapeutique.

Il est donc avantageux de proposer une utilisation du dispositif selon l'invention comme moyen de localisation de la zone limitée in-vivo, pour laquelle le plan est une table sur laquelle, en tant que sujet, un être vivant est allongé et la zone limitée est un ensemble de cellules in-vivo à localiser spatialement au moyen de coordonnées spatiales instantanément délivrées par la sonde à ultrasons et de moyen de calcul de la position instantanée de ladite sonde par rapport à un repère tridimensionnel lié au plan.

Cette utilisation du dispositif selon l'invention peut prévoir que la sonde est couplée à une unité de contrôle apte à une visualisation ou/et un traçage spatial dynamique de l'ensemble des cellules in-vivo, la dite unité étant pilotée par un opérateur ou un algorithme de reconnaissance. Ceci favorise la recherche et le traçage de la zone voulue et ses limites.

Ladite unité de contrôle peut être également couplée à un mécanisme additionnel de commande forcée de positionnement de la sonde sur un axe d'orientation principal, autour duquel la sonde est mise en libre orientation, le dit mécanisme étant piloté par l'opérateur ou l'algorithme de reconnaissance. Ce mécanisme de pilotage forcé peut servir à positionner initialement la sonde ou à corriger une position de la sonde lors d'un diagnostic ou une thérapie annexe afin d'améliorer la vision des cellules.

Enfin, l'utilisation du dispositif selon l'invention prévoit enfin une fonction d'asservissement sécuritaire et précis de tout appareillage annexe au dispositif afin de le guider spatialement et dynamiquement, en ce que l'unité de contrôle est reliée à une unité de commande de positionnement POS_BEAM, CTRL d'une installation par exemple radio-thérapeutique dont la cible est positionnable sur la zone limitée in-vivo vers laquelle le champ principal de vision de la sonde à ultrasons est maintenue dirigée.

## Revendications

1. Dispositif de maintien d'au moins une sonde (E) à ultrason adaptée à la visualisation d'une zone limitée d'un sujet mobile et déformable (P),
comprenant un support mobile (SE) dans et par rapport à une ouverture (S_{T}) d'un plan (T) contre lequel une partie superficielle du sujet est appuyée, le dit support mobile maintenant solidairement au moins une partie émettrice/réceptrice de la sonde de façon à la rendre librement mobile par diverses poussées mécaniques exercées par des pressions variables provenant de la partie superficielle en direction de l'ouverture.

2. Dispositif selon revendication 1, pour lequel au moins un film de gel acoustique est disposé entre la sonde et la partie superficielle afin d'évacuer un interstice d'air sur un canal d'ultrasons entre la sonde et la partie superficielle.

3. Dispositif selon revendication 1, pour lequel la partie émettrice/réceptrice de la sonde est alimentée par un flux de gel acoustique (G) provenant d'un réservoir de distribution dudit gel permettant une canalisation directe des ultrasons entre la sonde et la partie superficielle, ladite canalisation étant rendue libre d'interstice d'air.

4. Dispositif selon revendication 1, pour lequel la partie émettrice/réceptrice de la sonde est recouverte d'une enceinte de gel acoustique (G) à géométrie variable assurant l'appui de la partie superficielle et permettant une canalisation directe des ultrasons entre la sonde et la partie superficielle, ladite canalisation étant rendue libre d'interstice d'air.

5. Dispositif selon revendication 1, pour lequel au moins une surface de l'ouverture comprend une enceinte de gel acoustique (G) à géométrie variable assurant l'appui de la partie superficielle et permettant une canalisation directe des ultrasons entre la sonde et la partie superficielle, ladite canalisation étant rendue libre d'interstice d'air.

6. Dispositif selon une des revendications 4 ou 5, pour lequel l'enceinte est une forme élastomère à géométrie variable et ayant une impédance acoustique minimisant des pertes acoustique entre une entrée/sortie d'ultrasons de la sonde et de la partie superficielle, idéalement par une valeur d'impédance avoisinée à celle de la partie superficielle.

7. Dispositif selon une des revendications précédentes, pour lequel par entrainement du support mobile, l'ouverture est positionnable sur au moins une dimension du plan.

8. Dispositif selon une des revendications précédentes, pour lequel le support mobile (SE) est inclinable autour d'une normale au plan.

9. Dispositif selon une des revendications précédentes, pour lequel le support mobile (SE) comprend des moyens de détermination de sa position en translation et orientation dans un référentiel tridimensionnel lié au plan.

10. Utilisation du dispositif selon une des revendications précédentes comme moyen de localisation de la zone limitée in-vivo, pour laquelle le plan est une table sur laquelle, en tant que sujet, un être vivant est allongé et la zone limitée est un ensemble de cellules in-vivo à localiser spatialement au moyen de coordonnées spatiales instantanément délivrées par la sonde et de moyen de calcul de la position instantanée de la sonde par rapport à un repère tridimensionnel lié au plan.

11. Utilisation selon revendication 10, pour laquelle la sonde est couplée à une unité de contrôle apte à une visualisation ou/et un traçage spatial de l'ensemble des cellules in-vivo, la dite unité étant pilotée par un opérateur ou un algorithme de reconnaissance.

12. Utilisation selon revendication 11, pour laquelle l'unité de contrôle est couplée à un mécanisme additionnel de commande forcée de positionnement de la sonde sur un axe d'orientation principal, autour duquel la sonde est mise en libre orientation, le dit mécanisme étant piloté par l'opérateur ou l'algorithme de reconnaissance.

13. Utilisation selon revendication 11 ou 12, pour laquelle l'unité de contrôle est reliée à une unité de commande de positionnement d'une installation radiothérapeutique dont la cible est positionnable sur la zone limitée in-vivo.
